(19) **Europäisches Patentamt**
**European Patent Office**
**Office européen des brevets**

(11) **EP 2 965 766 B1**

(12) **EUROPEAN PATENT SPECIFICATION**

(45) Date of publication and mention
of the grant of the patent:
**12.04.2017 Bulletin 2017/15**

(51) Int Cl.:
*A61L 2/04* *(2006.01)*     *A61L 2/10* *(2006.01)*
*C02F 1/02* *(2006.01)*     *C02F 1/32* *(2006.01)*
*A23L 3/28* *(2006.01)*

(21) Application number: **14382273.2**

(22) Date of filing: **11.07.2014**

(54) **System and method for sterilizing a fluid**

System und Verfahren zum Sterilisieren einer Flüssigkeit

Système et procédé de stérilisation d'un fluide

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**13.01.2016 Bulletin 2016/02**

(73) Proprietors:
• **Salas Vicente, Fidel**
**12412 Geldo (ES)**
• **Salas Vicente, José**
**12412 Geldo (ES)**
• **Guamis Alegre, Alex**
**08012 Barcelona (ES)**
• **Guamis Alegre, David**
**08012 Barcelona (ES)**
• **Moreta Bufill, Leo**
**08008 Barcelona (ES)**

(72) Inventors:
• **Salas Vicente, Fidel**
**12412 Geldo (ES)**
• **Salas Vicente, José**
**12412 Geldo (ES)**
• **Guamis Alegre, Alex**
**08012 Barcelona (ES)**
• **Guamis Alegre, David**
**08012 Barcelona (ES)**
• **Moreta Bufill, Leo**
**08008 Barcelona (ES)**

(74) Representative: **Carvajal y Urquijo, Isabel et al
Clarke, Modet & Co.
Suero de Quiñones, 34-36
28002 Madrid (ES)**

(56) References cited:
**EP-A2- 0 202 820**     **US-A1- 2002 096 648**
**US-A1- 2003 052 278**     **US-A1- 2008 210 884**

EP 2 965 766 B1

**Description**

**Field of the invention**

The present invention relates to a system for processing fluids for sanitization and/or sterilization thereof by means of the controlled sequential and/or simultaneous application of germicidal ultraviolet radiation (preferably type C) and temperature, which may range from cooling to moderate (preferably) and high temperatures of products such as foods, ingredients, perfumes, scents, cosmetics, pharmaceutical products (medicines), hospital products (sera, haematological fluids, etc.), chemicals, products containing alcohol, petroleum derivatives, etc., and the like.

**Background of the invention**

A problem to be solved in the industry consists of sanitizing or sterilizing fluids (foods, ingredients, perfumes, scents, cosmetics, pharmaceutical products (medicines), hospital fluids (sera, haematological fluids, etc.), chemicals, products containing alcohol, petroleum derivatives etc., and the like), which due to their specific characteristics, are sensitive to specific thermal treatments, thereby suffering a loss of quality, at the functional or technological level (foaming, emulsifying, colouring, thickening, texturizing capacities, etc.), at the nutritional level (loss of vitamins, aminoacids and essential fatty acids, polyphenols, antioxidants, etc.) or at the organoleptic level (loss of scents, flavours, colours, textures, etc.).

UVC light has been applied in the purification and treatment of water as a low-cost, safe alternative for improving taste and odour. UVC light has a bactericidal effect on microorganisms. Although it is considered a bactericide, it affects all the types of microscopic organisms (viruses, bacteria, algae, fungi, yeasts and protozoa). The disinfecting power of UVC light is the result of its action on cellular DNA, causing a decrease in their respiratory activity, blocking the processes of synthesis and inhibiting or retarding mitosis. On the other hand, the effect of UVC light on two contiguous thymine or cytosine bases (pyrimidines) in the same strand of DNA or RNA generates the respective dimers, preventing DNA or RNA replication in the microorganisms, and thus their reproduction.

The bactericidal action of UVC light depends primarily on the dose applied to the microorganism, and the same is dependent on the irradiance and application time. Irradiance is the potency of UV light per unit area measured in microwatts per square centimetre. The corresponding dose is the product of the irradiance per application time and it is expressed in millijoules per square centimetre or the equivalent in microwatts per second per square centimetre.

Another feature of UVC light is that its bactericidal effect is cumulative over time (dose).

UV radiation minimizes the formation of chemicals and/or by-products. This process is also dry, cold, and requires little maintenance and has lower running costs compared with conventional thermal processes, wherein energy consumption is very high. On the other hand, because these are cold or moderate temperature processes, the unwanted effects of conventional thermal treatments are prevented. For these reasons, there is a growing interest in using UVC light for disinfecting foods. However, every food, cosmetic, pharmaceutical product, etc., has its own composition and this may determine that required UVC doses are different between them. A disadvantage of using UVC light for sanitizing is the low transmittance of the majority of fluids, which entails that only thin layers near the emitter may be treated efficiently.

The low transmittance of UVC light in most liquids other than water is well known. The effect of UVC light penetration depends on the type of liquid, the absorbance at the specified wave length, solutes present in the liquid and suspended material. Increasing the amount of solids will reduce the penetration intensity of UVC radiation, just as, large suspended particles may also block the incidence of light on the microbial load. For example, UV light penetration in juices ranges between several hundreds of microns and a few millimetres, due to a 90 % absorption of light caused by their compounds or suspended particles.

In certain foods or technological processes it might be necessary to reduce or inactivate certain enzymes, as for example, in vegetable or fruit juices, the inactivation of Pectin-Methyl-Esterase (PME), which causes loss of texture, Peroxidase (PO), catalysing oxidation processes, or Poly-Phenol-Oxidase (PFO) which has a role in darkening or browning reactions, oxidizing phenolic compounds to quinones. With respect to the effect of UVC on the enzymes, in general they cause some degree of inactivation, in accordance with the dose and matrix in which they are found.

At present there are different basic types of UV emitters available, of which the emitters based on mercury vapour may be highlighted.

In terms of killing germs, low pressure emitters are often the most widely used in the industry, since they offer an almost monochromatic emission that is condensed in 254 nm, very close to the absorption maximum of the DNA molecule at 260 nm and with easy handling and manipulation. Compared to medium pressure and high pressure emitters, low pressure emitters have better performance (30 - 40 % versus the 10 - 15 % of medium pressure) and work at more moderate temperatures (40 °C - 110 °C versus 600 °C - 900 °C), although the UV potency emitted per wave length unit is much lower. Document US 2008/210884 A discloses a device for irradiating a liquid.

Description of the invention

The present invention provides a system for sterilizing a fluid, comprising a UV emitter (1) surrounded by an elongated element (2) arranged concentrically with respect to the UV emitter, through which a coolant fluid cooling the UV emitter circulates, the elongated element (2) being surrounded by an elongated element (3) arranged concentrically with respect to the UV emitter, through which the fluid to be sterilized circulates, the elongated element (3) being surrounded by an elongated element (4), arranged concentrically with respect to the UV emitter, through which a coolant or heating fluid cooling or heating the fluid to be sterilized circulates, hereinafter system of the invention.

In the present specification, the term "fluid" is a liquid or gas.

One embodiment is the system of the invention, wherein the elongated element (3) through which the fluid to be sterilized circulates has coarse walls or spiral pathways.

This makes that the fluid to be sterilized circulate in a turbulent and non-laminar manner, to ensure that entire fluid volume is subjected to the same treatment or dose of UVC dose and temperature.

In the present invention, the thickness of the layer of fluid to be treated may even be lower than 1 mm. The back pressure to which the fluid is subjected (by means of a back pressure valve) together with the turbulence promoters, make the fluid to be treated circulate in a turbulent flow manner during processing thereof even in such tight spaces.

Different bibliographical sources suggest that it is necessary for all parts of the fluid to be exposed to at least a dose of 400 J/m2 (40 mJ/cm2) of UVC light (254 nm) to ensure an adequate 5 Log reduction of a target or marker microorganism, to obtain (in the case of food industry) a microbiologically safe food. Considering that said dose of UVC should be applied to the entire system to ensure that the liquid fluid is treated equally.

In the present invention, said dose or higher doses may be reached at the end of the treatment, since the system is designed so that an indefinite number of systems may be coupled together in a series. Also, the intensity of cutting-edge lamps (both excimer and mercury arc emitters), which are capable of giving said dose in just a few seconds of treatment, makes it so that said dose may be easily reached. Moreover, with regulation of the flow rate (the speed at which the fluid will circulate through the system) by means of the impeller pump, it is possible to increase the treatment time and therefore the dose. The present invention factors in the possibility that, should it be necessary to reduce the flow rates through the systems, groups of systems may be coupled together in parallel, in order to prevent the reduction of the total production flow rate (L/hour) of the equipment.

Another embodiment is the system of the invention, wherein the fluid to be sterilized is selected from the group consisting of a food, a cosmetic, a drug, a pharmaceutical composition, hospital fluid, a scent, a perfume and a chemical compound.

Another embodiment is the system of the invention, wherein the UV emitter emits a wave length of between 200 and 312 nm.

Another embodiment is the system of the invention, wherein the distance between the external wall of the elongated element (2) and the internal wall of the elongated element (3) is between 0.5 and 5 mm.

Another embodiment is the system of the invention, wherein one or more systems may be coupled together in series or in parallel. In particular, one of the systems has a UV emitter that emits at a wave length of between 290 and 320 nm.

The present invention also optionally factors in the possibility of using one or several (an assembly) UVB systems, with the same design as the UVC systems, and which therefore may be coupled together in series or in parallel, and placed after the UVC systems. The main purpose of the UVC systems would be to apply longer wave length treatments to repair or rebuild certain compounds, which depending on the composition of the fluid to be treated, might be degraded or react (primarily by oxidation) with other compounds of the fluid itself, slightly (but perhaps noticeably) changing the biochemical, organoleptic and/or nutritional characteristics of the treated fluid, and in the case of foods, for example, bringing about a loss of quality in said food. The use of said step is to be optional, because utilization thereof will be highly influenced by the desired expectations of the final product, as well as of the final composition of the product (fats, proteins, sugars, etc.).

Another embodiment is the system of the invention, comprising devices for measuring and controlling the emission irradiance of the UV emitter.

Another embodiment is the system of the invention, comprising devices for measuring and controlling the temperatures.

Since the UVC lamp that is found at the centre of the system and is hermetically insulated from the rest of the concentric tubes through which the fluid to be sterilized and the coolant and heating fluids circulate, the coatings forming the spaces wherein these fluids circulate require connector tubes (independent between both fluids) at the system outlets connecting to the inlets of the next system, to be used as a circulation system. The coolant or heating fluid passing through the system is re-circulated, whereas the fluid to be sterilized (e.g. food), even though it could also be re-circulated through the system, is preferably treated continuously by means of the groups of systems in series or in parallel, without being recirculated, unless a drop in any of the variables makes it so that the required dose is not reached, thus diverting the product (by means of a diverter valve) to the initial tank or receptacle for subsequent recirculation. A conditioning system for cooling/heating may be added at the inlet or outlet of the system to cool/heat the fluid to be treated before and after the UV light treatment.

The dose should be administered equally to the entire product volume, and as has been described, in the present invention this is achieved in the system of the invention. Different factors may help to achieve an equal dose in the entire product volume, for example: that the product has to be circulated through several (2 or more) systems in series or in parallel; that between these systems, the pipes (connecting the outlets to the inlets of the next one) have different section volumes than the section volumes in the segment of the system; that the geometrical and spatial arrangement of the systems forces the fluid or product to change its direction in the space (not the direction within the section through which the fluid circulates), either by placing horizontally or vertically (or alternating), direction changes from right to left or vice versa between systems (always taking into account that the outlet of one goes to the inlet of the other); that the circuit of fluid to be treated (product) may be counter-pressured by means of the back pressure valve (at the end of the circuit); that the presence of homogenizers at the outlet of a system forces the fluid to mix before the inlet of the next one; that the presence of turbulence promoters, in the chamber through which the fluid to be treated passes, increases turbulence and homogenization of the latter.

The possibility of applying different controlled temperatures at the same time as the UV are applied to the fluid to be treated and in such an efficient and homogeneous manner, may be useful for different applications such, for example:

- Obtaining synergistic microbial inactivation results, i.e. which exceed those that would be obtained if added together, applying the UVC and temperature separately.
- Obtaining total microbial inactivation for various kinds of microorganisms with varying degrees of sensitivity to ultraviolet light and the thermal process, thus enabling the use more moderate temperatures.
- Obtaining enzymatic inactivation, by means of applying moderate temperatures, for example of between 60-80 °C.
- Obtaining synergistic enzymatic inactivation results, which exceed the total of separate UVC and temperature treatments.
- Causing certain components (for example proteins) to stabilize in certain fluids at moderate temperatures.
- Facilitating the homogenization, dissolution and dispersion of various compounds in the fluids to be treated, as a result of the low or moderate temperatures.
- Encouraging the kinetics of certain chemical or enzymatic reactions using low or moderate temperatures, which benefit the final objectives of the fluid to be treated.
- The temperature and the control thereof may encourage the fluidity of the products or fluids having a certain level of viscosity, thereby facilitating the circulation thereof through the entire circuit.
- The temperature and the control thereof may facilitate cleaning processes in the circuit, once production has come to an end, since it helps to dissolve and drag residues of the treated fluids, and furthermore increases the effectiveness of cleaning products such as, for example, alkalis (NaOH at 3 % and 80 °C), acids (HNO$_3$ "nitric acid" at 2 % and 45-50 °C), commercial detergents, enzyme products, peracetic acid ($C_2H_4O_3$), hydrogen peroxide ($H_2O_2$), etc.
- The temperature and the control thereof may facilitate the disinfection and sterilization of the areas in the system when high temperatures are employed.

The present invention factors in various safety systems to prevent and detect problems of leaks and breakages between concentric tubes, and more specifically in those related to the UV emitters and the material surrounding this emitter, which is preferably quartz. Said safety systems provide higher safety regarding possible deviations of materials (quartz) to the fluids to be treated, damage to facilities by electrical shorts, safety for operators and, above all, provide a safety system of the pre-set conditions, both of UV dosage (intensity and time), temperature, and back pressure of the treatment, preventing the "poorly" treated fluid from getting packaged as correct. Such security measures may be listed in summary form as:

- Having temperature sensors along the circuit, and primarily (but not exclusively) at the outlet of the systems, which constantly send information about the temperature of the fluid to be treated to the control panel.
- Having sensors measuring the irradiance of non-ionizing radiation (UVC and/or UVB) inside each system and which constantly send information about the irradiance emitted by the emitter to the control panel.
- Having a thin electric track in the quartz of the emitter and/or protective quartz cover. Any breakage of the quartz would cause a breakage of the conductive track and therefore would cut the electrical current flow, signalling to the control panel, and the latter taking the actions programmed. Said conducting wire, preferably, but not exclusively, may be made of gold.
- Having a UV-transparent polymeric cover over the quartz which, should breakage occur, contains the splinters and prevents them from joining the fluid to be treated.
- Having gaskets providing air-tightness and sealing the junctions between the UV emitter and the quartz cover. Said gaskets are made with a design and materials (for example, but not limited to, fluorinated polymer) that tolerate the most extreme conditions of CIP-type (Clean in Place) cleaning and disinfecting and SIP (Steam in Place) sterilization, commonly used in food industries.
- Having a back pressure system (preferably a back pressure valve, although not exclusively) and exerting a constant pressure on the forward movement of the fluid to be treated, and thus helping to generate turbulent flow profiles.
- Having a "Control Panel" that actuates a diverter valve that diverts the fluid to be treated placed before the hygienic or aseptic packaging system (tank and packer), said conditioning of the diverter valve may be due to failure of any of the initially pre-set conditions, such as (preferably, but not limited to) the temperature, the UVC or UVB light intensity, treatment time (settings of the flow rate through the pumps impelling the product), breakage of the lamp or quartz cover, loss of product pressure, etc., which would keep product which has not been properly treated from being packaged (preferably, but not exclusively, that does not reach the reservoir or aseptic tank for further hygienic or aseptic packaging), such that the product could be diverted by means of the diverter valve to the initial tank for recirculation, or to any other tank in order to discard the product or otherwise.

The invention also provides a method for the sterilization of a fluid, which is carried out in a system comprising a UV emitter (1) surrounded by an elongated element (2) arranged concentrically with respect to the UV emitter through which a coolant fluid cooling the UV emitter circulates, the elongated element (2) being surrounded by an elongated element (3) arranged concentrically with respect to the UV emitter through which the fluid to be sterilized circulates, the elongated element (3) being surrounded by an elongated element (4) arranged concentrically with respect to the UV emitter through which a coolant or heating fluid cooling or heating the fluid to be sterilized circulates, which comprises subjecting the fluid to be sterilized to UV radiation at a temperature ranging between -20 °C y 160 °C.
Another embodiment is the method of the invention, wherein the temperature ranges between 2 and 80 °C.

**Brief description of the drawings**

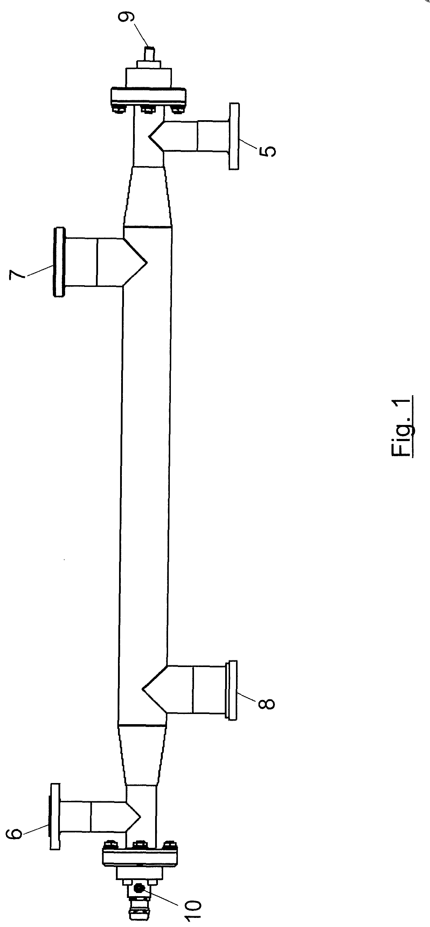

Figure 1. Shows an external side view of the system of the invention, wherein it is possible to observe the arrangement of the inlet and outlet points of the various fluid circuits: inlet (5) and outlet (6) of the fluid to be sterilized, inlet (7) and outlet (8) of the coolant or heating fluid, air inlet (9) and outlet (10).

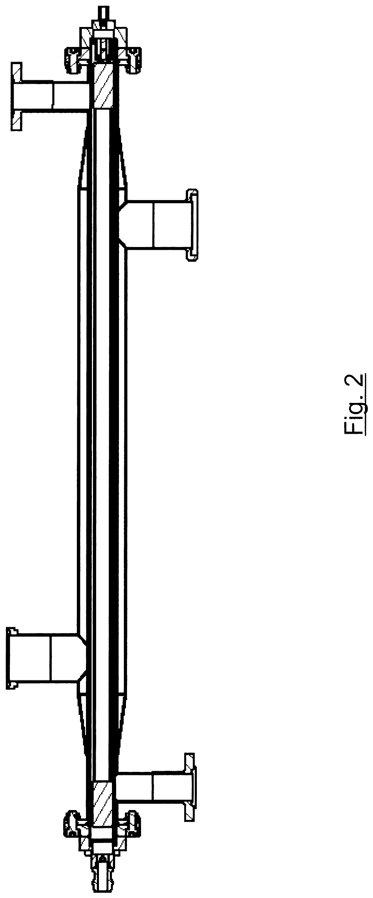

Figure 2. Shows a longitudinal section of the system of the invention, wherein it is possible to observe the arrangement of the various chambers through which the various fluids circulate, as well as the arrangement of the UV emitter.

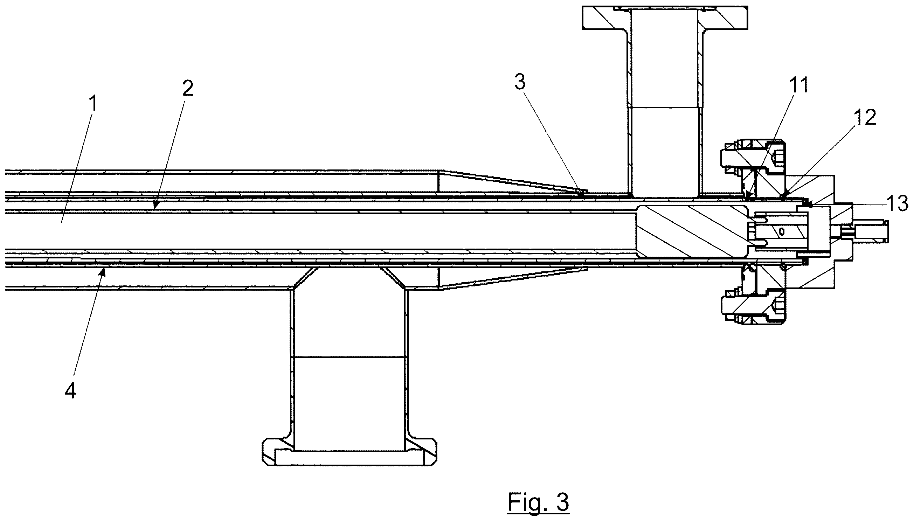

Figure 3. Shows an enlarged view of the longitudinal section, wherein it is possible to observe in greater detail the arrangement of the chambers through which the various fluids circulate: air (2), fluid to be sterilized (3), coolant or heating liquid (4), UV emitter (1). It is also possible to observe the arrangement of the gaskets or seals (11, 12, 13) that provide air-tightness between the emitter, the quartz cover and the chamber through which the product circulates.

**Preferred embodiments**

Example 1. Effect of UVC light treatment on lethality in different microorganisms.

By way of example to demonstrate the effectiveness of the present invention, various microorganisms having different levels of sensitivity to UVC treatment were studied in an aqueous solution having a low absorbance coefficient (0.43 cm$^{-1}$) and in liquid egg white having a high absorbance coefficient (107 cm$^{-1}$), at different temperatures, 20 and 50 °C in the treatment with inoculated aqueous solutions and at 20 and 55 °C in the treatments with inoculated liquid egg whites. The concentration of microorganisms in the control samples amounted to approximately $10^6$-$10^7$ cfu/ml. To calculate the dose received (Equation 1), it was necessary to calculate the transmitted Irradiance (*I*) by means of the Beer-Lambert Law (Equation 2) taking into account the thickness of the fluid, path length (for the aqueous solution and liquid egg white), which was 1 mm (d = 0.1 cm), the absorbance coefficients for $\lambda$ = 254 nm (described above) and incident Irradiance (*$I_0$* = 31 mW/cm$^2$). The microbiological results in Tables 1-3 are expressed as lethality (Equation 3).

$$Dose = Irradiance \cdot Time \text{ (Equation 1)}$$

In Equation 1, *Dose* is expressed in mW·S/cm$^2$, *Irradiance* is expressed in mW/cm$^2$ and *Time* is expressed in seconds.

$$I = I_0 \cdot 10^{-\varepsilon d} \text{ (Equation 2)}$$

In Equation 2, $I$ is transmitted Irradiance, $I_0$ is incident Irradiance, $\varepsilon$ is Absorbance coefficient and $d$ is path length.

$$Lethality = Log_{10}(N_0/N) \qquad \text{(Equation 3)}$$

In Equation 3, $N_0$ is the initial cfu/cm$^2$ number before the treatment and $N$ is the cfu/cm$^2$ number after the treatment.

Table 1. Effect of UVC light treatment with an irradiance of 31 mW/cm$^2$ at 20 °C for different exposure times on the lethality of different microorganisms in an aqueous solution (Absorbance coefficient: 0.43 cm$^{-1}$). Data from three independent experiments with duplicated quantification of the results of every experiment (n=6). The average $\pm$ standard deviation is shown. Lethality is expressed in cfu/cm$^2$.

| | Exposure time (seconds) | | | | | |
|---|---|---|---|---|---|---|
| | 3 s | 6 s | 12 s | 18 s | 24 s | 30 s |
| *A. niger* (spores) | 0.47 $\pm$ 0.27 | 1.12 $\pm$ 0.15 | 2.04 $\pm$ 0.05 | 3.58 $\pm$ 0.04 | 4.15 $\pm$ 0.17 | $\geq$ 5.1 |
| *B. subtilis* (spores) | 1.53 $\pm$ 0.55 | 2.9 $\pm$ 0.31 | 4.88 $\pm$ 0.31 | 5.91 $\pm$ 0.23 | $\geq$ 6.5 | --- |
| *S. aureus* | 3.43 $\pm$ 0.13 | 5.43 $\pm$ 0.64 | $\geq$ 6.7 | --- | --- | --- |
| *E. coli* | 4.83 $\pm$ 0.24 | 5.79 $\pm$ 0.96 | $\geq$ 6.9 | --- | --- | --- |
| *L. innocua* | 3.28 $\pm$ 0.28 | 4.93 0.65 | $\geq$ 6.8 | --- | --- | --- |
| *M. luteus* | 2.53 $\pm$ 0.21 | 5.32 $\pm$ 0.71 | $\geq$ 6.5 | --- | --- | --- |
| *P. fluorescens* | 2.71 $\pm$ 0.25 | 4.75 $\pm$ 0.53 | $\geq$ 7.1 | --- | --- | --- |

Table 2. Effect of UVC light treatment with an irradiance of 31 mW/cm$^2$ at 50 °C for different exposure times on the lethality of different microorganisms in an aqueous solution (Absorbance coefficient: 0.43 cm$^{-1}$). Data from three independent experiments with duplicated quantification of the results of every experiment (n=6). The average $\pm$ standard deviation is shown. Lethality is expressed in cfu/cm$^2$.

| | Exposure time (seconds) | | | | | |
|---|---|---|---|---|---|---|
| | 3 s | 6 s | 12 s | 18 s | 24 s | 30 s |
| *A. niger* (spores) | 0.43 $\pm$ 0.31 | 1.25 $\pm$ 0.13 | 2.28 $\pm$ 0.08 | 3.82 $\pm$ 0.21 | 4.86 $\pm$ 0.08 | $\geq$ 5.1 |
| *B. subtilis* (spores) | 1.31 $\pm$ 0.44 | 2.94 $\pm$ 0.38 | 4.82 $\pm$ 0.44 | 6.01 $\pm$ 0.33 | $\geq$ 6.5 | --- |
| *S. aureus* | 3.17 $\pm$ 0.15 | 6.33 $\pm$ 0.45 | $\geq$ 6.7 | --- | --- | --- |
| *E. coli* | 4.16 $\pm$ 0.22 | 6.9 | --- | --- | --- | --- |
| *L. innocua* | 3.07 $\pm$ 0.21 | 5.94 $\pm$ 0.75 | $\geq$ 6.8 | --- | --- | --- |
| *M. luteus* | 2.88 $\pm$ 0.36 | 5.14 $\pm$ 0.89 | $\geq$ 6.5 | --- | --- | --- |
| *P. fluorescens* | 3.58 $\pm$ 0.34 | 6.34 $\pm$ 0.44 | $\geq$ 7.1 | --- | --- | --- |

Table 3. Effect of UVC light treatment with an irradiance of 31 mW/cm$^2$ for different exposure times on the lethality of different microorganisms in liquid egg white (Absorbance coefficient: 107 cm$^{-1}$). Data from three independent experiments with duplicated quantification of the results of every experiment (n=6). The average $\pm$ standard deviation is shown. Lethality is expressed in cfu/cm$^2$.

| | Exposure time (seconds) | | | | | |
|---|---|---|---|---|---|---|
| | 3 s | 6 s | 12 s | 18 s | 24 s | 30 s |
| 20°C | | | | | | |
| B. subtilis (spores) | 0.21 ± 0.18 | 0.51 ± 0.21 | 1.12 ± 0.28 | 1.74 ± 0.12 | 2.4 ± 0.42 | 3.1 ± 0.54 |
| M. luteus | 0.5 ± 0.21 | 1.04 ± 0.33 | 1.96 ± 0.24 | 3.15 ± 0.23 | 4.01 ± 0.33 | 5.3 ± 0.64 |
| E. coli | 1.01 ± 0.61 | 1.62 ± 0.15 | 3.21 ± 0.33 | 5.02 ± 0.31 | 6.3 ± 0.25 | ≥ 6.4 |
| 55°C | | | | | | |
| B. subtilis (spores) | 0.19 ± 0.26 | 0.6 ± 0.63 | 0.98 ± 0.15 | 1.8 ± 0.41 | 2.51 ± 0.5 | 2.95 ± 0.58 |
| M. luteus | 0.66 ± 0.07 | 1.14 ± 0.21 | 2.14 ± 0.2 | 3.71 ± 0.05 | 4.68 ± 0.18 | 6.1 ± 0.15 |
| E. coli | 1.2 ± 0.23 | 1.88 ± 0.24 | 3.55 ± 0.54 | 5.88 ± 0.33 | ≥ 6.4 | --- |

Starting from the data expressed in the above tables (Table 1-3) it has been observed that lethality increases linearly and proportionally at longer exposure times, at least in the time ranges which have been tested.

When an Irradiance of 31 mW/cm$^2$ was applied, at 20 °C in aqueous solutions having a low absorbance coefficient (0.43 cm$^{-1}$) and times were between 3 and 6 seconds, lethality levels (reductions) of between 2.5-5.8 Logarithmic units (Log) were reached in vegetative bacteria, whereas the more ultraviolet-resistant microorganisms (*B. subtilis* spores and *A. niger* spores) reached reductions of 0.5-3 Log.

When an Irradiance of 31 mW/cm$^2$ was applied, at 20 °C in fluids having a high absorbance coefficient (107 cm$^{-1}$), such as liquid egg white, and times were between 6 and 18 seconds, the reductions were 1.6-5, 1-3 and 0.5-1.7 Log for the vegetative bacteria (*E. coli* and *M. luteus*) and *B. subtilis* spores, respectively.

When an irradiance of 31 mW/cm$^2$ was applied, at 50 °C in aqueous solutions having a low absorbance coefficient (0.43 cm$^{-1}$), and times were between 3 and 6 seconds, lethality levels (reductions) of between 3 and ≥6.9 Log were reached in vegetative bacteria, whereas in the sporulated microorganisms (*B. subtilis* spores and *A. niger* spores) reductions of 0.4-3 Log were achieved, nearly the same as when room temperature was applied (20 °C).

However, when an Irradiance of 31 mW/cm$^2$ was applied, at 55 °C in fluids having a high absorbance coefficient (107 cm$^{-1}$), such as liquid egg white, and times were between 6 and 18 seconds, the reductions were 2-6, 1-3,7 and 0,6-1,8 Log for the vegetative bacteria (*E. coli* and *M. luteus*) and *B. subtilis* spores, respectively.

Furthermore, the liquid egg white (LEW) samples treated with or without UVC and/or temperature, never coagulated and conserved the main functional characteristics (colour, odour, viscosity, foaming capacity, etc.).

**Claims**

1. A system for sterilizing a fluid, comprising a UV emitter (1) surrounded by an elongated element (2), arranged concentrically with respect to the UV emitter, through which a coolant fluid cooling the UV emitter circulates, the elongated element (2) being surrounded by an elongated element (3), arranged concentrically with respect to the UV emitter, through which the fluid to be sterilized circulates, **characterised in that** the elongated element (3) is surrounded by an elongated element (4), arranged concentrically with respect to the UV emitter, through which a coolant or heating fluid cooling or heating the fluid to be sterilized circulates.

2. The system according to claim 1, **characterized in that** the elongated element (3) through which the fluid to be sterilized circulates has coarse walls or spiral pathways.

3. The system according to one of the claims 1 or 2, **characterized in that** the fluid to be sterilized is selected from the group consisting of a food, a cosmetic, a drug, a pharmaceutical composition, a hospital fluid, a scent, a perfume and a chemical compound.

4. The system according to any of the claims 1 to 3, **characterized in that** the UV emitter emits at a wave length of between 200 and 312 nm.

5. The system according to the claims 1 to 4, **characterized in** the distance between the external wall of the elongated element (2) and the internal wall of the elongated element (3) is between 0.5 and 5 mm.

6. The system according to any of the claims 1 to 5, **characterized in that** two or more systems may be coupled together in series or in parallel.

7. The system according to claim 6, **characterized in that** one of the systems has a UV emitter that emits at a wave length of between 290 and 320 nm.

8. The system according to any of the claims 1 to 7, **characterized in that** it comprises devices for measuring and controlling the emission irradiance of the UV emitter.

9. The system according to any of the claims 1 to 8, **characterized in that** it comprises devices for measuring and controlling the temperatures.

10. A method for the sterilization of a fluid, the method being carried out in a system comprising a UV emitter (1) surrounded by an elongated element (2) arranged concentrically with respect to the UV emitter through which a coolant fluid cooling the UV emitter circulates, the elongated element (2) being surrounded by an elongated element (3) arranged concentrically with respect to the UV emitter through which the fluid to be sterilized circulates, **characterised in that** the elongated element (3) is surrounded by an elongated element (4) arranged concentrically with respect to the UV emitter through which a coolant or heating fluid cooling or heating the fluid to be sterilized circulates, and **in that** it comprises subjecting the fluid to be sterilized to UV radiation at a temperature ranging between -20 °C and 160 °C.

11. The method according to claim 10, **characterized in that** the temperature ranges between 2 and 80 °C.

**Patentansprüche**

1. System zum Sterilisieren einer Flüssigkeit, umfassend einen UV-Strahler (1) umgeben von einem verlängerten Element (2), welches in Bezug auf den UV-Strahler konzentrisch angeordnet ist, durch welches eine Kühlflüssigkeit, welche den UV-Strahler kühlt, fließt, wobei das verlängerte Element (2) von einem verlängerten Element (3) umgeben ist, welches in Bezug auf den UV-Strahler konzentrisch angeordnet ist, durch welches die zu sterilisierende Flüssigkeit fließt, **dadurch gekennzeichnet, dass** das verlängerte Element (3) von einem verlängerten Element (4) umgeben ist, welches in Bezug auf den UV-Strahler konzentrisch angeordnet ist, durch welches eine Kühl- oder Heizflüssigkeit, welche die zu sterilisierende Flüssigkeit kühlt oder erwärmt, fließt.

2. System nach Anspruch 1, **dadurch gekennzeichnet, dass** das verlängerte Element (3), durch welches die zu sterilisierende Flüssigkeit fließt, raue Wände oder spiralförmige Bahnen aufweist.

3. System nach einem der Ansprüche 1 oder 2, **dadurch gekennzeichnet, dass** die zu sterilisierende Flüssigkeit aus der Gruppe bestehend aus einem Nahrungsmittel, einem Kosmetikum, einem Arzneimittel, einer pharmazeutischen Zusammensetzung, einer Flüssigkeit zur krankenhäuslichen Verwendung, einem Duftstoff, einem Parfüm und einer chemischen Verbindung ausgewählt wird.

4. System nach einem der Ansprüche 1 bis 3, **dadurch gekennzeichnet, dass** der UV-Strahler bei einer Wellenlänge die zwischen 200 und 312 nm liegt strahlt.

5. System nach den Ansprüchen 1 bis 4, **dadurch gekennzeichnet, dass** der Abstand zwischen der Außenwand des verlängerten Elements (2) und der Innenwand des verlängerten Elements (3) zwischen 0,5 und 5 mm liegt.

6. System nach einem der Ansprüche 1 bis 5, **dadurch gekennzeichnet, dass** zwei oder mehr Systeme in Reihe oder parallel miteinander gekoppelt sein können.

7. System nach Anspruch 6, **dadurch gekennzeichnet, dass** eines der Systeme einen UV-Strahler aufweist, welcher bei einer Wellenlänge die zwischen 290 und 320 nm liegt strahlt.

**8.** System nach einem der Ansprüche 1 bis 7, **dadurch gekennzeichnet, dass** es Vorrichtungen zur Messung und Steuerung der gestrahlten Bestrahlungsstärke des UV-Strahlers umfasst.

**9.** System nach einem der Ansprüche 1 bis 8, **dadurch gekennzeichnet, dass** es Vorrichtungen zur Messung und Steuerung der Temperaturen umfasst.

**10.** Verfahren zur Sterilisierung einer Flüssigkeit, wobei das Verfahren in einem System durchgeführt wird, umfassend einen UV-Strahler (1) umgeben von einem verlängerten Element (2), welches in Bezug auf den UV-Strahler konzentrisch angeordnet ist, durch welches eine Kühlflüssigkeit, welche den UV-Strahler kühlt, fließt, wobei das verlängerte Element (2) von einem verlängerten Element (3) umgeben ist, welches in Bezug auf den UV-Strahler konzentrisch angeordnet ist, durch welches die zu sterilisierende Flüssigkeit fließt, **dadurch gekennzeichnet, dass** das verlängerte Element (3) von einem verlängerten Element (4) umgeben ist, welches in Bezug auf den UV-Strahler konzentrisch angeordnet ist, durch welches eine Kühl- oder Heizflüssigkeit, welche die zu sterilisierende Flüssigkeit kühlt oder erwärmt, fließt, und dadurch, dass es das Aussetzen der zu sterilisierenden Flüssigkeit zu UV-Strahlung bei einer Temperatur die zwischen -20°C und 160°C liegt umfasst.

**11.** Verfahren nach Anspruch 10, **dadurch gekennzeichnet, dass** die Temperatur zwischen 2 und 80°C liegt.

## Revendications

**1.** Système de stérilisation d'un fluide comprenant un émetteur UV (1) entouré d'un élément allongé (2), disposé concentriquement par rapport à l'émetteur UV, à travers lequel circule un fluide de refroidissement refroidissant l'émetteur UV, l'élément allongé (2) étant entouré d'un élément allongé (3), disposé concentriquement par rapport à l'émetteur UV, à travers lequel circule le fluide à stériliser, **caractérisé en ce que** l'élément allongé (3) est entouré d'un élément allongé (4), disposé concentriquement par rapport à l'émetteur UV, à travers lequel circule un fluide de refroidissement ou de réchauffement refroidissant ou réchauffant le fluide à stériliser.

**2.** Système selon la revendication 1, **caractérisé en ce que** l'élément allongé (3) à travers lequel circule le fluide à stériliser a des parois épaisses ou des trajectoires spirales.

**3.** Système selon l'un des revendications 1 ou 2, **caractérisé en ce que** le fluide à stériliser est choisi parmi le groupe composé d'un aliment, un cosmétique, un médicament, une composition pharmaceutique, un fluide d'hôpital, un parfum et un composé chimique.

**4.** Système selon l'une quelconque des revendications 1 à 3, **caractérisé en ce que** l'émetteur UV émet une longueur d'onde allant de 200 à 312 nm.

**5.** Système selon les revendications 1 à 4, **caractérisé en ce que** la distance entre la paroi externe de l'élément allongé (2) et la paroi interne de l'élément allongé (3) varie entre 0,5 et 5 mm.

**6.** Système selon l'une quelconque des revendications 1 à 5, **caractérisé en ce que** deux ou plusieurs systèmes peuvent être couplés ensemble en série ou en parallèle.

**7.** Système selon la revendication 6, **caractérisé en ce que** un des systèmes a un émetteur UV qui émet à une longueur d'onde allant de 290 à 320 nm.

**8.** Système selon l'une quelconque des revendications 1 à 7, **caractérisé en ce qu'**il comprend des dispositifs pour mesurer et contrôler l'éclairement d'émission de l'UV.

**9.** Système selon l'une quelconque des revendications 1 à 8, **caractérisé en ce qu'**il comprend des dispositifs pour mesurer et contrôler les températures.

**10.** Procédé de stérilisation d'un fluide, le procédé étant mis en oeuvre dans un système comprenant un émetteur UV (1) entouré d'un élément allongé (2) disposé concentriquement par rapport à l'émetteur UV, à travers lequel circule un fluide de refroidissement refroidissant l'émetteur UV, l'élément allongé (2) étant entouré d'un élément allongé (3) disposé concentriquement par rapport à l'émetteur UV à travers lequel circule le fluide à stériliser, **caractérisé en ce que** l'élément allongé (3) est entouré d'un élément allongé (4) disposé concentriquement par rapport à l'émetteur UV à travers lequel circule un fluide de refroidissement ou de réchauffement refroidissant ou réchauffant le fluide à stériliser, et **en ce qu'**il comprend le fait de soumettre le fluide à stériliser à un rayonnement UV à une température allant de -20°C à 160°C.

11. Procédé selon la revendication 10, **caractérisé en ce que** la température varie de 2 à 80°C.

Fig. 1

Fig. 2

Fig. 3

EP 2 965 766 B1

**EP 2 965 766 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2008210884 A